# EUROPEAN PATENT SPECIFICATION

(11) **EP 2 793 823 B1**
(45) Date of publication and mention of the grant of the patent: **06.12.2017**
(21) Application number: 12806808.7
(22) Date of filing: 11.12.2012
(51) Int. Cl.: A61K 8/37, A61K 8/34, A61K 8/06, A61Q 17/04

(54) **SUNCARE FORMULATIONS AND METHODS**
SONNENSCHUTZFORMULIERUNGEN UND VERFAHREN
PRÉPARATIONS ANTISOLAIRES ET PROCÉDÉS

(30) Priority: 22.12.2011 US 201161579054 P
(43) Date of publication of application: 29.10.2014
(73) Proprietor: Dow Global Technologies LLC, Midland, MI 48674 (US); Rohm and Haas Company, Philadelphia, PA 19106 (US)
(72) Inventor: PIECHOCKI, Christian, F-67500 Marienthal (FR); TULCHINSKY, Michael, L., Midland, MI 48642 (US); DE LA TORRE, Frederic, Midland, MI 48674 (US)
(74) Representative: Houghton, Mark Phillip
(86) International application number: PCT/US2012/068901
(87) International publication number: WO 2013/095995

(56) References cited:
- DE-A1-102009 050 430
- US-A- 5 362 494
- US-A1- 2010 129 299
- US-B1- 6 207 140

## Description

### Field

The present invention relates to suncare compositions. Methods for making, and uses for the same are disclosed.

### Background

One of the most challenging aspects of suncare composition formulation is ensuring that, once applied to a person, the active ingredient remains on the skin after exposure to water or perspiration. On the other hand, the composition must be capable of being removed by conventional soaps or other cleansers. In the past, one strategy was to incorporate film forming ingredients into sunscreen lotions and creams.

However, most of the film formers that are suitable for sunscreen lotion and creams are not compatible for alcohol-based sunscreen spray products, due to solubility/stability issues that will cause spray hardware (for example, the valve and actuator, or pump) to eventually clog. Moreover, even for formulations where this is not a concern, there are cultural reasons for avoiding alcohols in certain geographies.

Accordingly, a suncare composition that is alcohol-free would be advantageous. Moreover, said suncare composition should be transparent, and not cause an undesirable white appearance when applied to the skin.

US 6207140 B1 discloses sun protection compositions comprising a monoglyceride (ether) sulfate. DE 10 1009 050430 discloses sunscreen emulsions comprising glycerol ethers.

US 2010/129299 discloses sunscreen emulsions comprising ethylhexylglycerin.

### Detailed Description

In one embodiment, the present invention provides a suncare composition, comprising a transparent aqueous microemulsion of a suncare active and a diglycerol ether of Formula I: 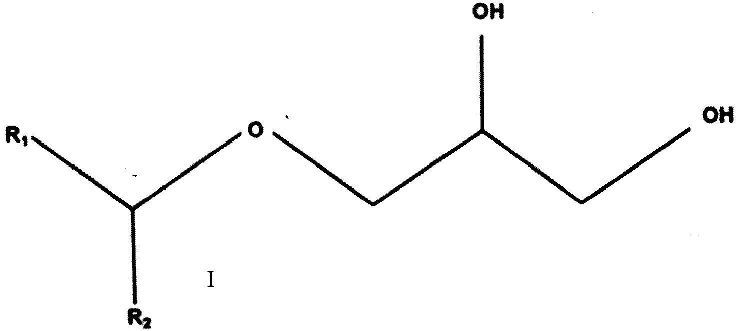 wherein:
R₁ is H or methyl; and
R₂ is CH(OH)-CH₂-O-R₃, wherein R₃ is C₆-C₈ alkyl.

A "microemulsion" as the term is used herein denotes a pseudo one-phase transparent mixture of two immiscible fluids. Microemulsions are thermodynamically stable and form spontaneously. Microemulsions are transparent or translucent, and do not display the opalescence of standard emulsions. The particle size of the resulting droplets is small enough so the resulting mixture is optically clear or translucent. Microemulsion droplet sizes are variously defined in the art with a droplet size typically below 0.14 micron.

"Suncare compositions" relates to compositions containing a suncare active to be topically applied to a person (including mouth, ear, and nasal cavities, but not ingested). Suncare compositions include lotions, creams and sprays, and are preferably sunscreens, more preferably with an SPF greater than 25. Such compositions must be cosmetically acceptable, that is, contain ingredients typically used in personal care compositions, and this is intended to underscore that materials that are toxic when present in the amounts typically found in personal care compositions are not contemplated as part of the present invention.

In one embodiment, the suncare active is at least one of octyl methoxycinnamate, avobenzone, para aminobenzoic acid, homosalate, titanium dioxide, zinc oxide, benzophenones, benzylidenes, salicylates, or other known UV filters.

In one embodiment, the suncare active is an organic pigment particulate. "Organic pigment particulate" refers to carbon-based particles of less than 250 nm, preferably less than 200 nm. In one embodiment, the organic pigment particulates are bisoctrizole. Bisoctrizole (INCI name: methylene bis-benzotriazolyl tetramethylbutylphenol) is a benzotriazole based organic compound which acts as a broad spectrum UV absorber, effective in both UVA and UVB ranges. Bisoctrizole is available from Ciba Specialty Chemicals under the tradename TINOSORB M. In one embodiment, the organic pigment particulates are present in an amount from 0.1% to 50% by weight of the composition, and more preferably 1% to 25%. In a most preferred embodiment, the organic pigment particulates are present in an amount constituting 10% active.

In one embodiment, the suncare composition further contains inorganic metal oxide sunscreen particles selected from zinc oxide (ZnO), titanium dioxide (Ti02), or mixtures thereof. In one embodiment, the inorganic metal oxide sunscreen particles are pigment grade zinc oxide or pigment grade titanium dioxide. In one embodiment, the inorganic metal oxide sunscreen particles are transparent zinc oxide or transparent titanium dioxide. Most inorganic metal oxide sunscreen particles used in a sunscreen produce a cosmetically undesirable white appearance caused by light scattering. Thus, the term "transparent inorganic metal oxide sunscreen particles," as used herein has a special meaning, referring to inorganic metal oxide sunscreen particles produced by a variety of processing conditions which render the inorganic metal oxide compositions as clear, or transparent, upon application. In other words, these specially processed inorganic metal oxide compositions do not appear white once applied, hence the moniker of transparent. Examples of transparent zinc oxide are disclosed in, for example, US Patent Nos. 5,223,250, 5,372,805, 5,573,753, 5,587,148, and 5,876,688. One example of a transparent zinc oxide is commercially available under the tradename Z-COTE from BASF Corporation (Germany). Another example of transparent zinc oxide is commercially available under the tradename ZINCLEAR IM from Antaria Limited (Australia). Another example of transparent zinc oxide is commercially available under the tradename Z-CLEAR from Actifirm (USA). Examples of transparent titanium dioxide are disclosed in, for example, US Patent Nos. 5,573,753, 5,733,895, and 7,390,355. Examples of transparent titanium dioxide are commercially available under the tradenames TIPAQUE and TTO-51(A) from Ishihara Sangyo Kaisha, Ltd. (Japan). Another example of a transparent titanium dioxide is commercially available under the tradename T-COTE from BASF Corporation (Germany). Another example of transparent titanium dioxide is commercially available under the tradename UFTR from Miyoshi Kasei (Japan). Another example of transparent titanium dioxide is commercially available under the tradename SOLAVEIL CLARUS from Uniqema (Great Britain). In one embodiment, the transparent inorganic metal oxide sunscreen particles are selected from transparent zinc oxide, titanium dioxide, or mixtures thereof.

The microemulsion composition will advantageously include from 1% by weight (wt. %) to 50 wt. %, preferably from 1% by weight (wt. %) to 30 wt. %, and most preferably 2 wt. % to 30 wt.% of such suncare actives. The weight ratio between the suncare active and the surfactant, if present in the composition of the present invention, generally is from 0.2:1 to 10:1, preferably from 0.5:1 to 6:1, more preferably from 1:1 to 5:1, and most preferably from 2:1 to 4:1.

In one embodiment, the suncare composition is ethanol-free. "Ethanol-free" means substantially free of ethanol, i.e., less than 1 wt %, and more preferably zero weight percent. The diglycerol ether is a compound of Formula (I). In one embodiment, R₂ is CH(OH)-CH₂-O-R₃, and R₃ is heptyl or octyl.

Compounds of the Formula I may be synthesized by catalytic reductive etherification in a known manner.

The amount of the compound of Formula I present in the microemulsion is generally from 1% by weight to 50 wt. %, preferably from 1 wt. % to 25 wt. %, more preferably from 3 wt. % to 20 wt. %, and most preferably from 5 wt. % to 15 wt. %.

In one embodiment, the microemulsion further comprises an aqueous medium, preferably water. The amount of aqueous medium present in the composition is generally from 50 wt. % to 95 wt. %, preferably from 55 wt. % to 90 wt. % and more preferably from 60 wt. % to 85 wt. %.

In one embodiment, the microemulsion further comprises one or more surfactants. Cationic, anionic, non-ionic, zwitterionic, amphophilic, or polymeric surfactants, and mixtures thereof may be used. Preferred surfactants include mixtures of non-ionic surfactants and anionic surfactants, mixtures of non-ionic surfactants and cationic surfactants, and mixtures of non-ionic surfactants and zwitterionic surfactants. Examples of particularly preferred surfactants are ethyloxalated alkanes, fatty acids, fatty acid salts, sulfonates or quaternary ammonium salts, and especially polyoxyethylene fatty ether surfactants, stearic acid and stearic acid salts, most preferably the sodium salt of stearic acid, sodium lauryl sulfate (SLS), sodium laureth sulfate (SLES), lauryl trimethyl ammonium chloride, Brij 30 (trademark of Uniqema, Chemical Abstracts name Poly(oxy-1,2-ethanediyl), alpha-dodecyl-omega-hydroxy-(9CI), Registery Number 9002-92-0), cetyl trimethyl ammonium chloride, or combinations thereof. In one embodiment, the surfactants are a mixture of non-ionic surfactants and anionic surfactants in a ratio of from 65:35 to 35:65, preferably 60:40.

The amount of surfactant which may be used in the composition of the present invention is generally from 0 wt. % to 50 wt. %, preferably from 0 wt. % to 20 wt. %, more preferably from 0 to 15 wt.% and most preferably from 0 to 10 wt. %; and when the surfactant is present in the composition, the amount of surfactant is preferably from 0.1 wt. % to 15 wt. %, and more preferably from 0.5 wt. % to 10 wt. %.

In one embodiment, the microemulsion further comprises contain one or more additional ingredients, such as fragrances, antioxidants, chelating agents, preservatives, thickening agents, cosmetic active ingredients, moisturizers, humectants, emollients, opacifiers, pearly gloss impacting substances, pigments, colorants, dyes and antifoams. The additional ingredients are generally present in the composition of the present invention from 0 wt % to 5 wt %, preferably from 0.5 wt % to 3 wt %, and more preferably from 0.1 wt % to 1 wt %.

The microemulsion perfumed aqueous cosmetic composition according to the present invention may be prepared, for example, by simple mixing of all the ingredients; for example by hand stirring or if need be by using a mechanical mixer (i.e. by some mechanically agitating means), the components of the present invention, and any optional components, to form a homogeneous mixture. The components of the present invention may be added together into a suitable reaction vessel and mixed in any order, using conventional processes well known to those skilled in the art. The microemulsion may be produced at room temperature or at an elevated temperature, for example up to 90°C, preferably up to 55°C can be employed. Microemulsions can be formulated to be optically clear, and stable from 5°C to 55°C.

### Examples

### Example 1

Suncare microemulsions of the present invention are described in TABLE 1 in grams:

**TABLE 1**

| | **Batch A** | **Batch B** | **Batch C** | **Batch D** | **Batch E** |
|---|---|---|---|---|---|
| BRIJ 97 nonionic surfactant | 0.27 | 0.27 | 0.27 | 0.27 | 0.27 |
| AOT 75 anionic surfactant | 0.24 | 0.24 | 0.24 | 0.24 | 0.24 |
| Hexenyl *cis-3* Salicylate | 0.75 | 0.75 | 0.75 | 0.75 | 0.75 |
| Water | 2.99 | 2.99 | 2.99 | 2.99 | 2.99 |
| 3-(penthyloxy)-1,2-propanediol | q.s. transparent | - - | - - | - - | - - |
| 3-(2-methylbutoxy)-1,2-propanediol | | q.s. transparent | - - | - - | |
| 3-[2-hydroxy-3-(heptyloxy) propoxy]-1, 2-propanediol | - - | - - | q.s. transparent | - - | |
| 3-[2-hydroxy-3-(2-ethylhexyloxy) propoxy]-1, 2-propanediol | - - | - - | - - | q.s. transparent | - - |
| 3-(butoxy)-1, 2-propanediol | - - | - - | - - | - - | q.s. transparent |

The components are mixed until a transparent microemulsion forms, usually requiring on average 0.75g of a compound of Formula I. The Suncare active : Surfactant ratio is 1.667, and the ratio of nonionic to anionic surfactant is 60:40. Please note that only Batch C and Batch D are according to the invention and that Batch A, Batch B and Batch E are outside the scope of the present invention,

### Example 2

A suncare microemulsion of the present invention is described in TABLE 2 in grams:

**TABLE 2**

| | **Batch C**' |
|---|---|
| Cremophor RH 40 nonionic surfactant | 0.27 |
| AOT 75 anionic surfactant | 0.24 |
| Hexenyl *cis-3* Salicylate | 0.75 |
| Water | 2.99 |
| 3-[2-hydroxy-3-(heptyloxy) propoxy]-1, 2-propanediol | q.s. transparent |

The components are mixed until a transparent microemulsion forms, usually requiring on average 0.75g of a compound of Formula I. The Suncare active : Surfactant ratio is 1.667, and the ratio of nonionic to anionic surfactant is 60:40.

### Example 3 - Comparative

A comparative alkyl diol microemulsion is described in TABLE 3 in grams:

**TABLE 3**

| | **Comparative Batch 1** | **Comparative Batch 2** |
|---|---|---|
| BRIJ 97 nonionic surfactant | 0.27 | - - |
| Cremophor RH 40 nonionic surfactant | - - | 0.27 |
| AOT 75 anionic surfactant | 0.24 | 0.24 |
| Hexenyl *cis*-3 Salicylate | 0.75 | 0.75 |
| Water | 2.99 | 2.99 |
| 1,2 Hexane Diol | q.s. transparent ∼0.75g | q.s. transparent ∼0.75g |

The components are mixed until a transparent microemulsion forms.

### Example 4

Compositions substantially according to Examples 1-3 were made and compared for solvent efficiency, the amount of specified compound of Formula I required to make a transparent microemulsion in comparison to the amount of 1,2 Hexane Diol required to make Comparative Batch 1. Batches A, B, C, and D were all found to more than 50% more efficient than the Comparative Batch 1. However, Batch D forms a gel. Batch E failed to form a microemulsion.

Batch C' was 73% more efficient than the Comparative Batch 2.

### Example 5

Suncare microemulsions of the present invention were made as described above with 3-[2-hydroxy-3-(heptyloxy) propoxy]-1,2-propanediol (from Batch C and Batch C' above) and a variety of suncare actives. Pure salicylates are easily microemulsified at about 5, 10 and 15% with 3-[2-hydroxy-3-(heptyloxy) propoxy]-1,2-propanediol. Whereas the concentration of sunscreen active is tripled (from 5 to 15%), the amount of cosolvent required is almost constant in all three formulations at around 10% in the microemulsion.

Various blends of at least two of homosalate, octisalate, oxybenzone, octinoxate, 4-methyl benzylidene camphor, octocrylene, avobenzone, bemotrizimol, ethyl hexyl triazone, and DHHB were microemulsified with varying levels of 3-[2-hydroxy-3-(heptyloxy) propoxy]-1,2-propanediol required, varying from 7-19%. Not all combinations formed microemulsions, but each compound was successfully microemulsified at least once in some combination.

## Claims

1. A suncare composition, comprising a transparent aqueous microemulsion of a suncare active and a monoalkyl glycerol ether or a diglycerol ether of Formula I: 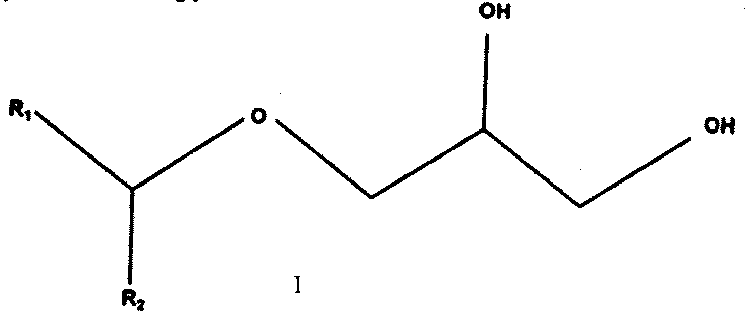 wherein:
R₁ is H or methyl; and
R₂ is CH(OH)-CH₂-O-R₃, wherein R₃ is C₆-C₈ alkyl.

2. The suncare composition of Claim 1, wherein the suncare active is homosalate, octisalate, oxybenzone, octinoxate, 4-methyl benzylidene camphor, octocrylene, avobenzone, bemotrizimol, ethyl hexyl triazone, and DHHB.

3. The suncare composition of Claim 1, wherein the suncare active is at least one of the salicylates.

4. The suncare composition of Claim 1, wherein the suncare composition is ethanol-free.

5. The personal care composition of Claim 1, wherein R₂ is CH(OH)-CH₂-O-R₃, and R₃ is heptyl or octyl.

6. The personal care composition of Claim 1, wherein the diglycerol ether is 3-[2-hydroxy-3-(heptyloxy) propoxy]-1, 2-propanediol.

## Patentansprüche

1. Eine Sonnenschutzzusammensetzung, beinhaltend eine transparente wässrige Mikroemulsion eines Sonnenschutzaktivstoffs und einen Monoalkylglycerolether oder einen Diglycerolether der Formel I: 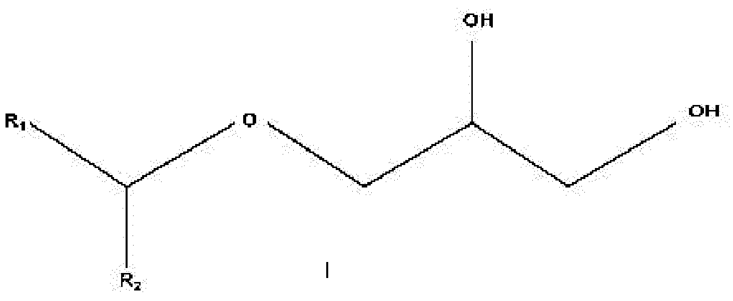 wobei
R₁ H oder Methyl ist; und
R₂ CH(OH)-CH₂-O-R₃ ist, wobei R₃ C₆-C₈-Alkyl ist.

2. Sonnenschutzzusammensetzung gemäß Anspruch 1, wobei der Sonnenschutzaktivstoff Homosalat, Octisalat, Oxybenzon, Octinoxat, 4-Methylbenzylidencampher, Octocrylen, Avobenzon, Bemotrizinol, Ethylhexyltriazon und DHHB ist.

3. Sonnenschutzzusammensetzung gemäß Anspruch 1, wobei der Sonnenschutzaktivstoff mindestens eines der Salicylate ist.

4. Sonnenschutzzusammensetzung gemäß Anspruch 1, wobei die Sonnenschutzzusammensetzung frei von Ethanol ist.

5. Körperpflegezusammensetzung gemäß Anspruch 1, wobei R₂ CH(OH)-CH₂-O-R₃ ist und R₃ Heptyl oder Octyl ist.

6. Körperpflegezusammensetzung gemäß Anspruch 1, wobei der Diglycerolether 3-[2-Hydroxy-3-(heptyloxy)propoxy]-1,2-propandiol ist.

## Revendications

1. Une composition de soin solaire, comprenant une microémulsion aqueuse transparente d'un actif de soin solaire et d'un éther de monoalkylglycérol ou d'un éther de diglycérol de Formule I : 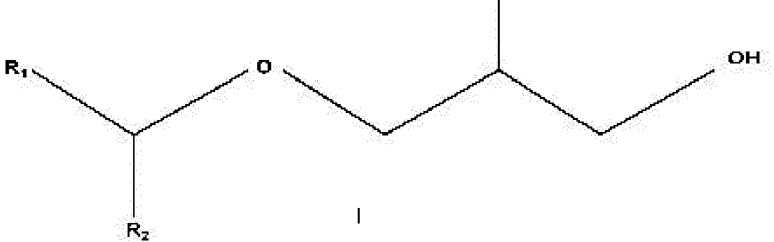 où :
R₁ est H ou un méthyle ; et
R₂ est CH(OH)-CH₂-O-R₃, où R₃ est un alkyle en C₆ à C₈.

2. La composition de soin solaire de la revendication 1, où l'actif de soin solaire est l'homosalate, l'octisalate, l'oxybenzone, l'octinoxate, le 4-méthylbenzylidène camphre, l'octocrylène, l'avobenzone, le bémotrizinol, l'éthylhexyle triazone, et le DHHB.

3. La composition de soin solaire de la revendication 1, où l'actif de soin solaire est au moins l'un des salicylates.

4. La composition de soin solaire de la revendication 1, la composition de soin solaire étant dépourvue d'éthanol.

5. La composition pour soins d'hygiène personnelle de la revendication 1, où R₂ est CH(OH)-CH₂-O-R₃, et R₃ est un heptyle ou un octyle.

6. La composition pour soins d'hygiène personnelle de la revendication 1, où l'éther de diglycérol est le 3-[2-hydroxy-3-(heptyloxy)propoxy]-1,2-propanediol.
